# EUROPEAN PATENT APPLICATION

(11) **EP 3 564 645 A1**
(43) Date of publication of application: **06.11.2019**
(21) Application number: 18170760.5
(22) Date of filing: 04.05.2018
(51) Int. Cl.: G01M 3/40, A61N 1/375

(54) **METHOD FOR TESTING PLASTIC ENCLOSURES OF MEDICAL DEVICES**

(71) Applicant: Dyconex AG, 8303 Bassersdorf (CH)
(72) Inventor: Bihler, Eckardt, 8406 Winterthur (CH)
(74) Representative: Keck, Hans-Georg

(57) **Abstract**

The present invention relates to a method for conducting a leak test of an enclosure (2), wherein the enclosure (2) comprises a plastic material, wherein a first electrode (10) is arranged with respect to the enclosure (2) such that it faces a portion (20) of the enclosure (2) and wherein a second electrode (11) is enclosed in said portion (20) of the enclosure (2), and wherein an electrolyte (4) comprising charged particles (5) is arranged outside the enclosure (1) between the first electrode (10) and the second electrode (11), and wherein a voltage is applied between the first and the second electrode (10, 11) such that charged particles (5) are moved towards the second electrode (11), and wherein an electrical current between the first and the second electrode (10, 11) is measured so as to determine whether charged particles (5) have diffused into the enclosure (1). Further, the invention relates to a device or assembly (1) that can be used for conducting the method according to the present invention.

## Description

The present invention relates to a method for conducting a leak test of an enclosure, particularly of an enclosure of a medical device, particularly of an implantable medical device. Furthermore, the invention relates to a medical device, particularly to an implantable medical device, that is particularly configured to be used with the method according to the present invention.

The present invention aims at testing the impermeability of enclosures of devices, particularly encapsulations, made from or comprising plastic, particularly polymeric, materials, with respect to liquids, particularly body fluids, particularly without destroying the product or negatively affecting the life span of the respective device.

Particularly, a frequently used leak test based on diffusion of helium (He) cannot be applied to enclosures comprising polymeric materials due to the fact that helium is adsorbed by such materials which heavily distorts the measurement of diffusion through a polymeric layer. Further, in order to get results regarding such soak tests within a reasonable time span, these tests have to be accelerated by using higher temperatures. However, mechanical properties of polymeric materials change (often in a non-linear fashion) with increasing temperatures (particularly in case of thermoplasts) such that no conclusion can be drawn regarding the expected life span of the device. Furthermore, the thermal acceleration reduces the life span of the device so that such soak tests can only be applied to test devices but not to actual products that are later offered on the market.

Therefore, the objective of the present invention is to provide a method for conducting a leak test that can be applied to a fully functional device without deteriorating the device.

This problem is solved by a method having the features of claim 1 as well as by a device having the features of claim 12.

Preferred embodiments of these aspects of the present invention are stated in the sub claims and are described below.

According to claim 1, a method for conducting a leak test of an enclosure, particularly of an enclosure of medical device, particularly of an implantable medical device, is disclosed, wherein the device can comprise components, such as electronic components, arranged in the enclosure, and wherein the enclosure comprises a plastic material (or is formed out of at least one or several plastic materials), wherein a first electrode is arranged with respect to the enclosure such that it faces a portion of the enclosure and wherein a second electrode is arranged inside the enclosure, and wherein an electrolyte comprising charged particles is arranged outside the enclosure between the first electrode and the second electrode, and wherein a voltage is applied to the first and the second electrode such that the charged particles are moved towards the second electrode, and wherein an electrical current between the first and the second electrode (due to charged particles diffusing into the enclosure and to the second electrode) is measured so as to determine whether charged particles of the electrolyte have diffused into the enclosure.

Furthermore, according to an embodiment, the enclosure is deemed not to leak in case said measured current is below a pre-defined threshold.

Particularly, the present invention uses for measuring impermeability of a plastic enclosure with respect to an electrolyte an acceleration of the latter due to an electrical field rather than using an acceleration caused by an increased temperature. For this, particularly, the enclosure/device to be tested is employed with structures that allow measurement of the electrolyte diffusion. Particularly, the functionality and the life span of the product/device are not tampered with by the measurement according to the present invention.

Due to the measurement principle/set-up according to the present invention, functional electronic devices and modules encapsulated with plastic, particularly polymeric, materials, can be tested regarding their impermeability to liquids (particularly electrolytes) within economically reasonable time spans. Thus, corresponding products that can be used as implants for the human or animal body can be produced and tested in an efficient manner.

Particularly, according to an embodiment of the method according to the present invention, said plastic material comprises or is formed by one of: a thermoplast, an elastomer, a polymer, parylene, silicone, a liquid crystal polymer.

Further, according to an embodiment, the respective plastic material is a biocompatible and/or biostable plastic material, particularly a biocompatible and/or biostable polymer (particularly LCP).

Furthermore, according to an embodiment of the method according to the present invention, the enclosure comprises a recess adjacent said portion of the enclosure, wherein said first electrode is arranged on a lateral wall of the recess such that it faces the second electrode, and wherein the electrolyte is arranged in said recess such that it is arranged between the first electrode and the second electrode.

Further, according to an embodiment of the method according to the present invention, the enclosure comprises a substrate for carrying components, such as electronic components, of the device and a lid bonded to said substrate at an interface between the substrate and the lid such that said components are encapsulated by the substrate and the lid.

Preferably, the lid is formed out of or comprises a plastic material, particularly one of: a thermoplast, an elastomer, a polymer, parylene, silicone, a liquid crystal polymer.

Preferably, the substrate is formed out of or comprises a plastic material, particularly one of: a thermoplast, a thermoset polymer, a polyimide, a liquid crystal polymer.

Further, according to an embodiment, the respective plastic material of the lid or substrate is a biocompatible and/or biostable plastic material, particularly a biocompatible and/or biostable polymer (particularly LCP).

Furthermore, according to an embodiment, the substrate can be a printed circuit board, particularly a multi-layered circuit board.

Further, according to an embodiment of the method according to the present invention, the recess extends (e.g. normal to the lid and normal the substrate) in the form of trench through the lid and into the substrate.

Further, in an embodiment of the method according to the present invention, the first electrode extends into the substrate and faces said interface between the lid and the substrate. Particularly, this is an advantageous configuration since the interface is most likely to be prone to leakages so that the first electrode can effectively cover this region of the enclosure.

Furthermore, according an embodiment of the method according to the present invention, the recess extends circumferentially, particularly in the form of a trench, along a periphery of the enclosure.

Furthermore, according to an embodiment of the method according to the present invention, the first electrode extends circumferentially along the recess.

Furthermore, according to an embodiment of the method according to the present invention, the second electrode also extends circumferentially along the recess albeit inside the enclosure, e.g. embedded between the lid and the substrate.

Further, according to an embodiment of the method according to the present invention, the first electrode forms an anode while the second electrode may form a cathode.

Further, the respective charged particle of the used electrolyte can be an ion, particularly a cation such as Cu⁺, or Li⁺. The electrolyte can be solution comprising such ions/cations (e.g. Cu⁺ or Li⁺).

According to a further embodiment of the method according to the present invention, the enclosure may comprise a plurality of recesses as well as a plurality of first and second electrodes.

Particularly, in an embodiment of the method, each second electrode is enclosed in a portion of the enclosure, wherein said portions and said recesses are arranged along the enclosure in an alternating fashion.

Further, particularly, each first electrode is arranged on a lateral wall of an associated recess such that it faces a second electrode arranged in an adjacent portion of the enclosure.

Furthermore, according to an embodiment of the method, an electrolyte is arranged in the respective recess such that it is arranged between a first electrode and a second electrode.

Further, in an embodiment of the method, a voltage is applied between the first electrodes and the second electrodes such that charged particles of the electrolyte are moved towards the respective second electrode, and wherein the second electrodes are connected in parallel to measure a resulting electrical current between the first and the second electrodes so as to determine whether charged particles have diffused into the enclosure.

Furthermore, yet another aspect of the present invention relates to a device. The device can be a medical device, particularly an implantable medical device. Further, the device can be an assembly of such a device. Particularly the medical device can be cardiac pacemaker.

The device comprises an enclosure that comprises a plastic material (or that is formed out of at least one or several plastic materials), wherein the enclosure comprises a recess that is configured to receive an electrolyte, wherein the recess comprises a lateral wall on which a first electrode is arranged such that it faces a portion of the enclosure, and wherein a second electrode is arranged in said portion of the enclosure such that it faces the first electrode.

Particularly, the recess is formed such that an electrolyte is arranged between the first electrode and the second electrode, when it resides in the recess.

Particularly, the plastic material of the enclosure can be one of the materials already stated above in conjunction with the method according to the present invention.

Furthermore, the device can comprise components, such as electronic components, encapsulated by the enclosure. Further, the enclosure can comprise a substrate for carrying electronic components of the device and a lid bonded to said substrate at an interface between the substrate and the lid such that said components comprised by the device are encapsulated by the substrate and the lid. Particularly, the lid and the substrate can be formed out of or comprise one of the materials already state above concerning the method pursuant to the present invention. Furthermore, the substrate can be a printed circuit board, particularly a multi-layered printed circuit board (see also above).

Furthermore, according to an embodiment of the device according to the present invention, the recess of the enclosure extends (e.g. normal to the lid and/or normal the substrate) through the lid into the substrate.

Furthermore, according to an embodiment of the device according to the present invention, the first electrode extends into the substrate and faces said interface between the lid and the substrate.

Furthermore, according to an embodiment of the device according to the present invention, the recess extends circumferentially, particularly in the form of a trench, along a periphery of the enclosure.

Furthermore, according to an embodiment of the device according to the present invention, the first electrode extends circumferentially along the recess.

Furthermore, according to an embodiment of the device according to the present invention, the second electrode extends circumferentially along the recess.

Furthermore, according to an embodiment of the device according to the present invention, the first electrode forms an anode while the second electrode forms a cathode.

Furthermore, the device or assembly having an open recess allowing access to said interface between the lid and the substrate may already be a fully functional device or assembly. However, the recess of the device or assembly can also be closed or covered, particularly by a plastic material and/or a metal (e.g. after having carried out the leak test).

Further features and embodiments of the present inventions will be described below with reference to the Figures, wherein
- Fig. 1: shows a cross-sectional view of a device according to the present invention for illustrating the method according to the present invention;
- Fig. 2: shows a top view of the enclosure shown in Fig. 1; and
- Fig. 3: shows a device/testing set-up using a plurality of first and second electrodes to measure diffusion of an electrolyte for conducting a leak test.

Particularly, the present invention relates to conducting leak tests of electronic devices 1 encapsulated with polymeric materials intended particularly for permanent implantation in the human or animal body. Devices 1 for implantation in the body known in the state of the art are encapsulated with a hermetic titanium enclosure. Here, helium leakage tests are used to test hermeticity. Polymer encapsulations, e.g. made of epoxy resin, can only be used for temporary implanted modules, since a clearly measurable diffusion of body fluid through the polymer encapsulation leads to corrosion of the components and vice versa pollutants from the encapsulated components enter the body.

Recent developments for the encapsulation of electronic devices 1 deal with biostable polymers such as parylenes and LCP (liquid crystal polymer). With these materials, a significantly better stability against diffusion of body fluids can be achieved. According to the state of the art, however, no suitable test methods are known. Using helium leak tests known for titanium enclosures fails for polymeric materials due to the strong absorption of helium from porous polymeric materials, which make the measurement of diffusion faulty. As an alternative, soak tests in saline solution or similar, also oxidative electrolytes are discussed for polymeric materials, which should come closest to the conditions in the body. In order to obtain results within an economically reasonable time span, the soak tests must be carried out at elevated temperatures in order to accelerate the process. However, it has been shown that the results of the soak tests are only of limited use at elevated temperatures, since the mechanical properties of the thermoplastics can change with temperature in such a way that other defects, e.g. delamination between boundary layers, which might not occur at body temperature, can be observed. Therefore, it is necessary to search for a method by means of which the diffusion of electrolytes along boundary layers / interfaces 24 can be accelerated. According to the present invention electrical fields are used to accelerate diffusion.

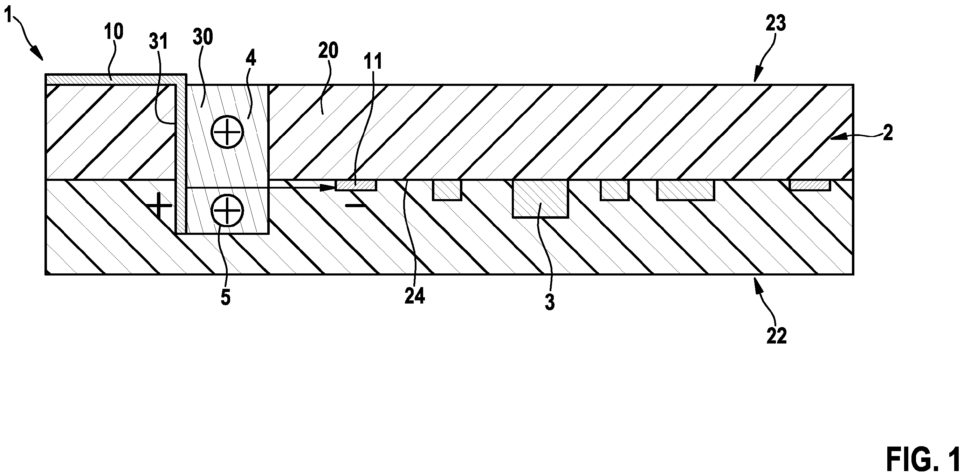

Fig. 1 schematically shows a cross-section of device 1 (e.g. an implantable medical device) according to an embodiment of the present invention, which device 1 comprises an enclosure 2 that can be formed by an e.g. multi-layered substrate 22 that can be formed out of or comprise a liquid crystal polymer, components 3 arranged thereon and/or incorporated therein, and an encapsulation with a polymeric lid 23 (e. g. LCP, parylene and/or silicone or other suitable materials) firmly attached to the substrate 22.

Further, a recess 30, e.g. in the form of trench is inserted at an edge of the lid 23 (e.g. by means of a laser), so that the recess 30 extends through the lid 23 into the substrate 22 and thus reveals a horizontal interface 24 running through the module/device 1. Diffusion along this interface 24 is the weakest point.

Later in the production process, this trench 30 can be closed by a suitable material such as a metallization in order to hermetically seal off this weak point as far as possible. For the purpose of measuring diffusion in the leak test according to the present invention, the recess 30 is not covered. Instead a first electrode 10 is provided on a lateral wall 31 of the recess opposing the portion 20 of the enclosure comprising said interface 24 so that the first electrode faces said portion 20/interface 24.

Alternatively, the first electrode 10 and the trench 30 can be removed from the device 1 after completion of the test.

This first electrode 10 / metallization can be formed either by a photolithographically defined physical vapor deposition (PVD) process or by means of an electrochemical coating.

To carry out the measurement, a suitable electrolyte is now filled into the recess. Salt solutions with e. g. Cu+ or Li+ ions are suitable for this purpose.

An electric field is applied between the first electrode 10 on the lateral wall 31 of the recess 30, which acts as an anode, and a second electrode that can be formed by a conductor strip embedded between the substrate 22 and the lid 23, which acts as a cathode.

Typical examples of dimensions are a recess (trench) width of 0.02mm to 0.1 mm and a distance of the second electrode 11 from the recess/trench of 0.02mm to 0.1 mm, so that field strengths of up to 250 kV/cm result with voltages between 100 Volt and 1000 Volt. This is still well below the expected penetration field strength of at least 1000 kV/cm (LCP). The current between the electrodes 10, 11 is measured using a suitable sensitivity. The expected insulation along the boundary layer is at least about 1012 Ohm, which would lead to a leakage current of about 1 nA at 1000 volts.

If the boundary layer of the substrate 22 is well fused to the lid 23, a resistivity in the order of the volume conductivity of 10¹⁸ Ohm (LCP) can be expected, which corresponds only to a leakage current of 10⁻¹⁵ A (or 10⁻³ pA) at 1000 volts. From the measured current over time (current integral = total charge = charge*particle amount). The mass flow can be estimated with using the geometric data of the space between the electrodes 10, 11 and can be calculated back to obtain the diffusion coefficients. With a specification for tolerable mass exchange over the service life, the maximum possible duration of use in the body can be estimated. For functional product testing, a maximum current value for a defined geometric arrangement of the electrodes can be used as a test criterion. In case this current is reached or exceeded, the enclosure is deemed to have a leak and can then be discarded.

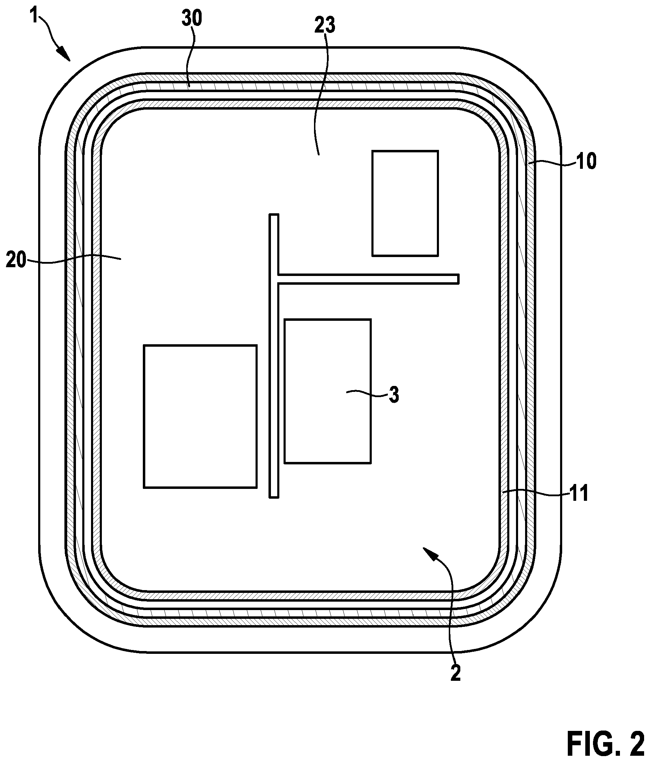

Particularly, as shown in Fig. 2 the recess / trench 30 can follow the course of an outer edge of the lid 23 of the already completed device / assembled module 1 (with lid 23 firmly attached to substrate 22). The second (counter) electrode (e.g. cathode) 11 can run along the recess 30 inside the enclosure 2. The anode 10 can extend along the trench 30 on an outer lateral side 31 of the trench 30 and has contact with the electrolyte. The electrodes can be connected from the outside via the production panel.

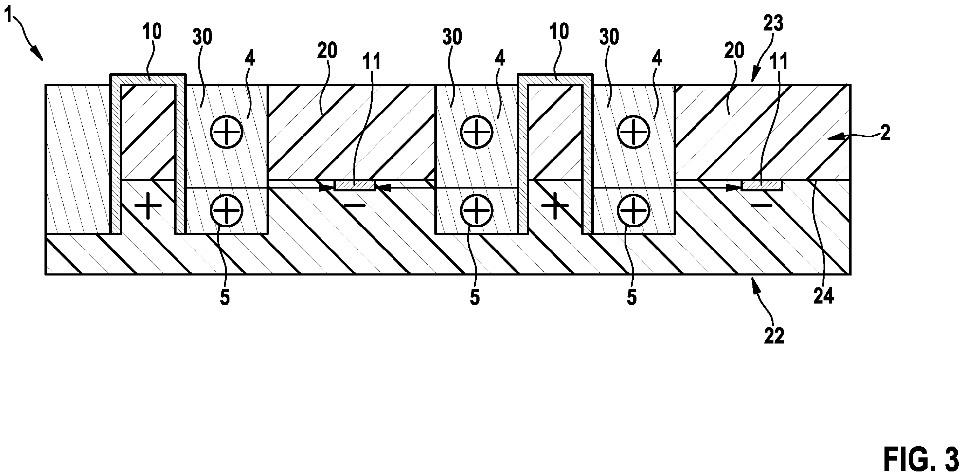

Higher sensitivity of the method that may be required for the characterization of materials and typical structures and the determination of test criteria, can be achieved by using several measuring cells in parallel. As shown in Fig. 3 each measuring cell can comprise an electrolyte-filled trench 30 and a buried second electrode 11. As a result, the electrode area available for measurement can be increased without changing the typical material structures and geometries.

Particularly, in such an embodiment, the enclosure 2 may comprise a plurality of recesses 30 as well as a plurality of first and second electrodes 10, 11.

Particularly, in an embodiment, each second electrode 11 is enclosed in a portion 20 of the enclosure 2, wherein said portions 20 and said recesses 30 are arranged along the enclosure 2 in an alternating fashion.

Further, particularly, each first electrode 10 is arranged on a lateral wall 31 of an associated recess 30 such that it faces a second electrode 11 arranged in an adjacent portion 20 of the enclosure 2. The respective electrolyte 4 is arranged in the respective recess 30 such that it is arranged between a first electrode 10 and a second electrode 11.

Also here, a voltage is applied between the first electrodes 10 and the second electrodes 11 such that charged particles 5 of the electrolyte 4 are moved towards the respective second electrode 11 due to a generated electric field, and wherein the second electrodes 11 are connected in parallel to measure a resulting electrical current between the first and the second electrodes 10, 11 so as to determine whether charged particles 5 have diffused into the enclosure 2.

## Claims

1. Method for conducting a leak test of an enclosure (2), wherein the enclosure (2) comprises a plastic material, wherein a first electrode (10) is arranged with respect to the enclosure (2) such that it faces a portion (20) of the enclosure (2) and wherein a second electrode (11) is enclosed in said portion (20) of the enclosure (2), and wherein an electrolyte (4) comprising charged particles (5) is arranged outside the enclosure (1) between the first electrode (10) and the second electrode (11), and wherein a voltage is applied between the first and the second electrode (10, 11) such that charged particles (5) are moved towards the second electrode (11), and wherein an electrical current between the first and the second electrode (10, 11) is measured so as to determine whether charged particles (5) have diffused into the enclosure (2).

2. Method according to claim 1, **characterized in that** said plastic material comprises or is formed by one of: a thermoplast, an elastomer, a polymer, parylene, silicone, a liquid crystal polymer.

3. Method according to claim 1 or 2, **characterized in that** the enclosure (2) comprises a recess (30) adjacent said portion (20) of the enclosure (2), wherein said first electrode (10) is arranged on a lateral wall (31) of the recess (30) such that it faces the second electrode (11), and wherein the electrolyte (4) is arranged in said recess (30) such that it is arranged between the first electrode and the second electrode (10, 11).

4. Method according to one of the preceding claims, **characterized in that** the enclosure (2) comprises a substrate (22) for carrying at least one electronic component (3) of the device (1) and a lid (23) bonded to said substrate (22) at an interface (24) between the substrate (22) and the lid (23) such that said at least one electronic component (3) is encapsulated by the substrate (22) and the lid (23).

5. Method according to claims 3 and 4, **characterized in that** the recess (30) extends through the lid (23) into the substrate (22).

6. Method according to claim 4 or 5, **characterized in that** the first electrode (10) extends into the substrate (22) and faces said interface (24) between the lid (23) and the substrate (22).

7. Method according to one of the claims 3 to 6, **characterized in that** the recess (30) extends circumferentially along a periphery of the enclosure (2).

8. Method according to one of the claims 3 to 7, **characterized in that** the first electrode (10) extends circumferentially along the recess (30).

9. Method according to one of the claims 3 to 8, **characterized in that** the second electrode (11) extends circumferentially along the recess (30).

10. Method according to one of the preceding claims, **characterized in that** the first electrode (10) forms an anode and/or that the second electrode (11) forms a cathode.

11. Method according to one of the preceding claims, **characterized in that** the respective charged particle (5) of the electrolyte (4) is one of: an ion, a cation, Cu⁺, Li⁺.

12. Medical device (1) comprising an enclosure (2) that comprises a plastic material, wherein the enclosure (2) comprises a recess (30) that is configured to receive an electrolyte (4), wherein the recess (30) comprises a lateral wall (31) on which a first electrode (10) is arranged such that it faces a portion (20) of the enclosure (2) and wherein a second electrode (11) is arranged in said portion (20) of the enclosure (2) such that it faces the first electrode (10).
